Europäisches Patentamt

European Patent Office

Office européen des brevets

(11) Veröffentlichungsnummer: **0 372 101**
**A1**

## (12) EUROPÄISCHE PATENTANMELDUNG

(21) Anmeldenummer: **88120176.8**

(22) Anmeldetag: **02.12.88**

(51) Int. Cl.5: **H05G 1/64, H04N 5/32**

(43) Veröffentlichungstag der Anmeldung:
**13.06.90 Patentblatt 90/24**

(84) Benannte Vertragsstaaten:
**DE FR GB**

(71) Anmelder: **Siemens Aktiengesellschaft**
**Wittelsbacherplatz 2**
**D-8000 München 2(DE)**

(72) Erfinder: **Gerlach, Hans-Jürgen, Dipl.-Ing.**
**Hofmannstrasse 5**
**D-8520 Erlangen(DE)**
Erfinder: **Aichinger, Horst, Dr. rer. nat.**
**Unterfarrnbacher Strasse 32**
**D-8510 Fürth(DE)**

(54) **Röntgendiagnostikanlage mit einer Bildverstärker-Fernsehkette.**

(57) Bei einer Röntgendiagnostikanlage mit Bildverstärker-Fernsehkette soll eine freie Dominantenwahl bei rascher Signalbildung erzielt werden.

Das Ausgangsbild des Röntgenbildverstärkers wird über eine Faseroptik (17) den lichtelektrischen Wandlern (18a usw.) zugeführt, die über eine Schaltvorrichtung (20) der gewünschten Form und Lage der Dominante entsprechend aktiviert werden. Die Anordnung mit den Wandlern (18a usw.) kann abnehmbar sein, so daß das Ausgangsbild des Röntgenbildverstärkers an der Ausgangsseite der Faseroptik (17) betrachtet werden kann.

FIG 2

EP 0 372 101 A1

## Röntgendiagnostikanlage mit einer Bildverstärker-Fernsehkette

Die Erfindung betrifft eine Röntgendiagnostikanlage mit einer Bildverstärker-Fernsehkette und Mitteln, die das Ausgangsbild des Röntgenbildverstärkers zusätzlich zur Fernsehkamera erfassen.

Die Mittel zur zusätzlichen Erfassung des Ausgangsbildes des Röntgenbildverstärkers können von einem Detektor für die mittlere Bildhelligkeit in einem vorbestimmten Bereich gebildet sein. Dieser Detektor kann aus einer Matrix von Fotoelementen bestehen, bei der entsprechend der gewünschten Dominante zur Dosisleistungsregelung und/oder zur automatischen Abschaltung einer Röntgenaufnahme eine bestimmte geometrische Anordnung der Fotoelemente zur Bildung eines Ausgangssignales aktiviert wird. Eine solche Matrix ist aufbaubedingt nur in einem festgelegten Modus, nämlich seriell abfragbar, was zu Einschränkungen in der Abfragegeschwindigkeit führt.

Der Erfindung liegt die Aufgabe zugrunde, eine Röntgendiagnostikanlage der eingangs genannten Art so auszubilden, daß eine Vielzahl von Meßfeldern mit variabler Form wählbar ist, so daß das jeweils gewählte Meßfeld optimal den jeweiligen Gegebenheiten anpaßbar ist, wobei eine hohe Abfragegeschwindigkeit ermöglicht ist.

Diese Aufgabe ist erfindungsgemäß gelöst durch eine Faseroptik, deren eines Ende das Bildverstärker-Ausgangsbild im telezentrischen Strahlengang zwischen Bildverstärker-Ausgangsschirm und Fernsehkamera aufnimmt und die dieses Bild weiterleitet.

Bei der erfindungsgemäßen Röntgendiagnostikanlage ist es möglich, den Fasern der Faseroptik auf der Ausgangsseite indivi duelle lichtelektrische Wandler zuzuordnen, so daß eine vorbestimmte Wandlerkonfiguration gebildet wird, bei der die Wandler parallel abgefragt und ihre Signale zu einem Gesamtsignal zusammengefaßt werden können.

Die Wandler mit der nachgeordneten Schaltung können abnehmbar mit der Faseroptik verbunden sein, so daß das Ausgangsbild des Röntgenbildverstärkers auf der Ausgangsseite der Faseroptik über eine Betrachtungsoptik betrachtet werden kann.

Die Erfindung ist nachfolgend anhand eines in der Zeichnung dargestellten Ausführungsbeispieles näher erläutert. Es zeigen:

Fig. 1 eine Röntgendiagnostikanlage nach der Erfindung,

Fig. 2 eine für die Erfindung wesentliche Einzelheit der Röntgendiagnostikanlage gemäß Fig. 1, und

Fig. 3 und 4 Varianten der Einzelheit gemäß Fig. 2.

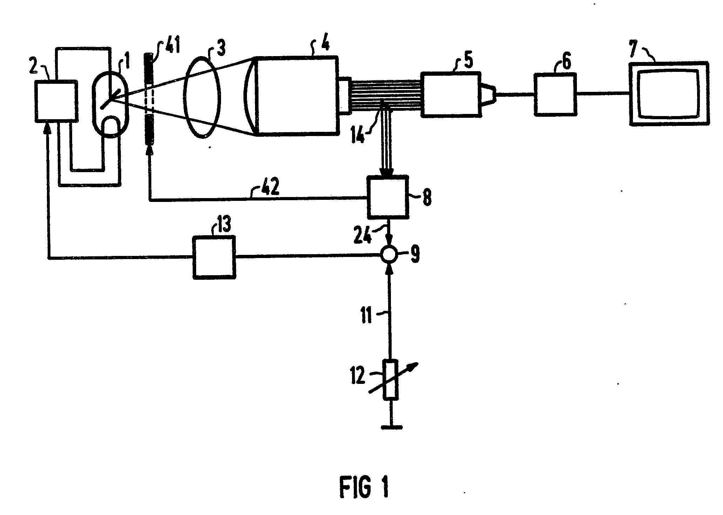

In der Fig. 1 ist eine Röntgenröhre 1 dargestellt, die von einem Röntgengenerator 2 gespeist wird. Ein Patient 3 wird von der Röntgenstrahlung durchstrahlt. Das Röntgenbild wird von einem Röntgenbildverstärker 4 verstärkt. Das am Ausgangsbildschirm des Röntgenbildverstärkers 4 erscheinende, verstärkte Röntgenbild wird von einer Fernsehkamera 5 aufgenommen und über eine Fernsehzentrale 6 auf einem Sichtgerät 7 wiedergegeben.

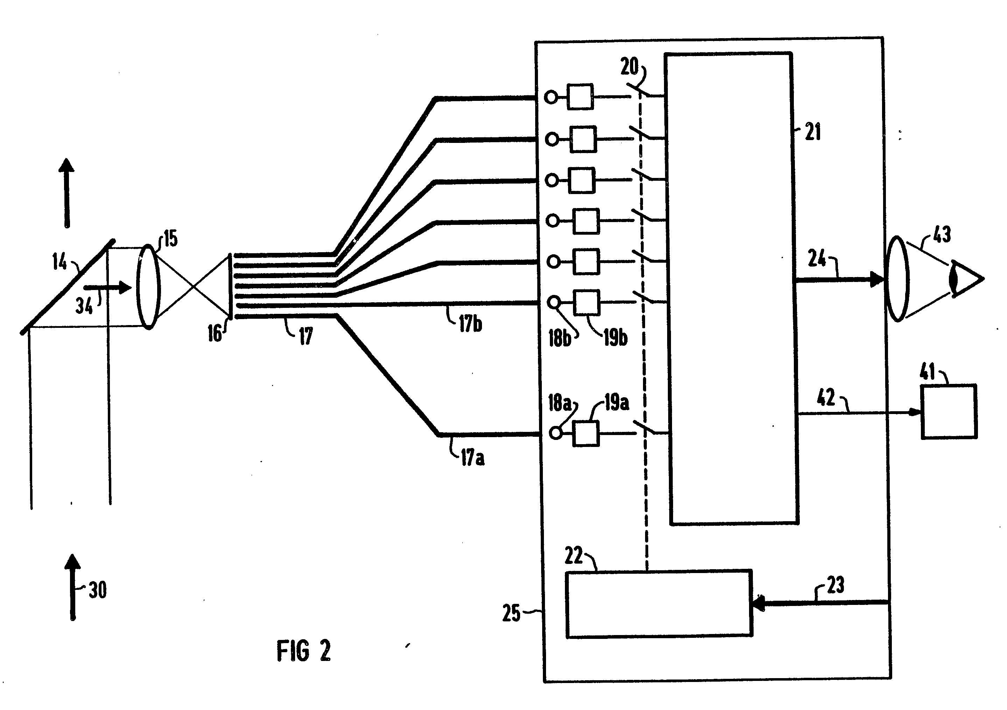

Zur Konstanthaltung der mittleren Bildhelligkeit in einem Meßfeld des Ausgangsschirmes des Röntgenbildverstärkers 4 ist der in der Fig. 2 näher dargestellte Detektor 8 als Istwertgeber vorgesehen, der ein entsprechendes Signal dem Istwerteingang eines Vergleichers 9 zuführt. Der Vergleicher 9 weist einen Sollwerteingang 11 auf, an dem ein dem Sollwert der mittleren Bildhelligkeit im Meßfeld des Ausgangsschirmes des Röntgenbildverstärkers 4 entsprechendes Signal liegt. In Abhängigkeit von der Differenz zwischen Ist- und Sollwert wird der Röntgen generator 2 von einer Helligkeitsregeleinrichtung 13 im Sinne eines Angleiches des Istwertes an den Sollwert beeinflußt. Für die Einstellung des Sollwertes ist ein Sollwertgeber 12 vorgesehen.

Dem Detektor 8 wird ein Teil des Lichtes des Röntgenbildverstärkers 4 mit Hilfe eines telezentrischen Spiegels 14 im Strahlengang zwischen dem Ausgangsleuchtschirm des Röntgenbildverstärkers 4 und der Fernsehkamera 5 zugeführt. Der Detektor 8 besitzt an seinem Eingang gemäß Fig. 2 eine Optik 15, die das Ausgangsbild des Röntgenbildverstärkers 4 in einer Ebene 16 darstellt, in der die Enden einer Faseroptik 17 mit einzelnen Lichtwellenleitern 17a, 17b usw. liegen. Den anderen Enden der Lichtwellenleiter 17a usw. sind lichtelektrische Wandler 18a, 18b usw. zugeordnet, die der jeweils empfangenen Helligkeit entsprechende elektrische Ausgangssignale liefern. Aus diesen Ausgangssignalen wird über Vorverstärker 19a, 19b usw., eine Schaltvorrichtung 20 und einen Signalformer 21 das Eingangssignal des Vergleichers 9 gebildet. Die Schalter der Schaltvorrichtung 20 werden durch eine Auswahlschaltung 22 angesteuert, der an ihrem Eingang 23 ein der gewünschten Dominante des Ausgangsbildes des Röntgenbildverstärkers 4 entsprechendes Signal zugeführt wird. Die Faseroptik 17 ist auf ihrer den Wandlern 18a usw. zugeordneten Ausgangsseite aufgefächert, so daß eine individuelle Zuordnung eines Wandlers 18a usw. zu dem jeweiligen Lichtwellenleiter 17a usw. möglich ist. Zur Dominantenbildung werden die der gewünschten Dominante entsprechenden Schalter der Schaltvorrichtung 20 geschlossen. Der Signalformer 21 bildet an seinem Ausgang 24 das dem Vergleicher 9 zugeführte, der mittleren Dosisleistung in der Dominante entsprechende Signal durch Zusammenfassung seiner Eingangssignale.

Die Anordnung gemäß Fig. 2 kann auch als Justierhilfe für die Einstellung der Lage der Dominante in bezug auf die Patientenebene dienen. Hierfür wird unter Röntgenstrahlung ein mit geeigneten Markierungen versehenes Phantom vor dem Röntgenbildverstärker 4 angeordnet und auf dem Ausgangsschirm des Rönt genbildverstärkers 4 und gleichzeitig in der Ebene 16 abgebildet. Durch Verschiebung der Eingangsfläche der Faseroptik 17 in der Ebene 16 erfolgt die Justierung. Hierzu ist die Anordnung 25 mit den Wandlern 18a usw. abnehmbar, so daß das Phantombild direkt an den Ausgangsenden der Lichtwellenleiter 17a usw. zu erkennen ist, wenn eine ausreichende Zahl davon verwendet wird. Dabei können auch zusätzlich passive Lichtwellenleiter ohne Wandler zu den aktiven Lichtwellenleitern mit Wandlern verwendet werden. Es ist auch möglich, einen Lichtwellenleiter-Faserstrang zu verwenden, der in der Ebene 16 aktive und passive Fasern enthält und sich dann in den aktiven Strang mit fest angebauten Wandlern und den passiven Strang ohne Wandler teilt. Während der aktive Strang aufgefächert wird, kann man den passiven Strang auf dem gleichen Gesamtdurchmesser wie in der Ebene 16 belassen. Er kann Aussparungen an den Stellen enthalten, die in der Ebene 16 von den aktiven Fasern eingenommen werden und wird an eine günstige Stelle im Gehäuse des Spiegels 14 geführt. Auch damit läßt sich die Lage der Meßfelder erkennen und justieren, nämlich indem die Phantommarkierungen mit den Lücken im Bild, das der passive Strang liefert, zur Deckung gebracht werden. Zur Sichtkontrolle muß gegebenenfalls eine Optik 43 zur Anpassung und Vergrößerung verwendet werden.

Bei dem Ausführungsbeispiel gemäß Fig. 1 dient das Ausgangssignal des Detektors 8 zur Dosisleistungsregelung. Es kann auch als Istwertsignal in einem Belichtungsautomaten zur automatischen Schaltung von Röntgenaufnahmen verwendet werden. Das Signal der Lichtwellenleiter, die den äußeren Rand des Bildes erfassen, kann auch so ausgewertet werden, daß damit ein Ausgangssignal auf der Leitung 42 erzeugt wird, mit dem eine Strahlenblende 41 zum Vermeiden von seitlichen Überstrahlungen angesteuert wird (Fig. 1).

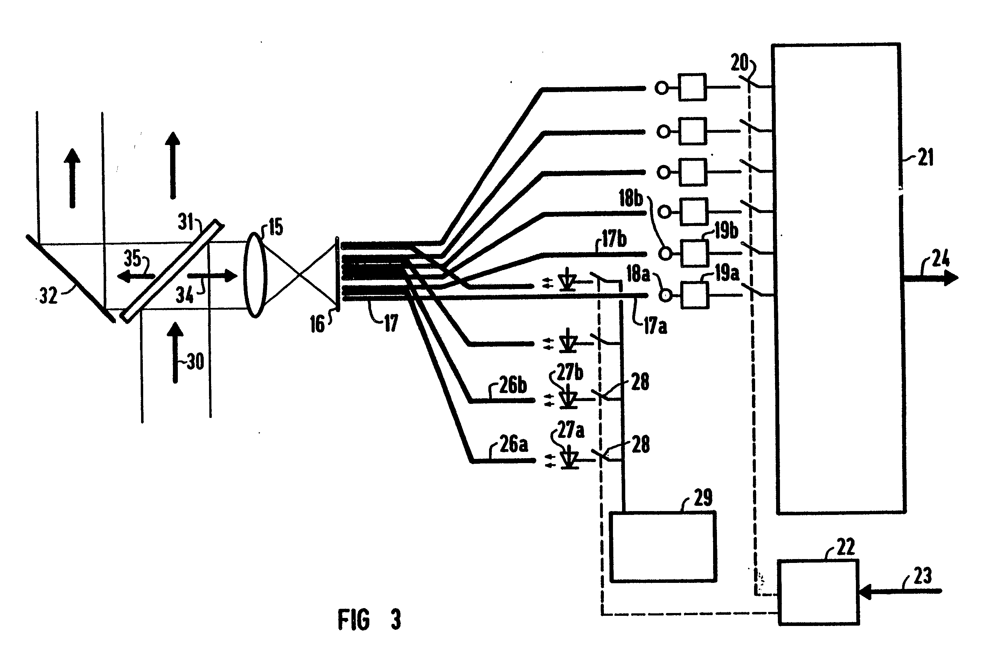

Die Variante der Anordnung gemäß Fig. 2, die in Fig. 3 dargestellt ist, besitzt zusätzlich zu den im Vergleich zur Fig. 2 mit den gleichen Bezugszeichen versehenen Komponenten passive Lichtwellenleiter 26a, 26b usw., die zur Rückprojektion der Dominante in den telezentrischen Strahlengang verwendet werden. Die in der Faseroptik 17 mit den aktiven Lichtwellenleitern 17a usw. zusammengefaßten Lichtwellenleiter 26a usw. werden je nach gewählter Dominante, d.h. je nach den mit Hilfe der Schaltvorrichtung 20 ausgewählten Wandlern 18a usw., mit einer entsprechenden Auswahl von Leuchtdioden 27a, 27b usw. beleuchtet. Hierzu werden die Leuchtdioden 27a usw. von der Auswahlschaltung 22 über eine Schaltvorrichtung 28 entsprechend angesteuert. Eine Lichtstärkensteuerung 29 erlaubt die Einstellung der Lichtstärke der Leuchtdioden 27a usw.

Das Licht vom Röntgenbildverstärker 4 kommt wie in Fig. 2 in Richtung des Pfeiles 30 an und wird durch einen teildurchlässigen Spiegel 31 teilweise wie in Fig. 2 in Richtung des Pfeiles 34 zur Ebene 16 gelenkt. Der Spiegel 31 läßt einen geringen Prozentsatz des auftreffenden Lichtes in Richtung zur Fernsehkamera 5 passieren. Das Ausgangslicht der Lichtwellenleiter 26a usw. gelangt durch die Optik 15 und den teildurchlässigen Spiegel 31 in Richtung des Pfeiles 35 auf den Spiegel 32, der es in Richtung zur Fernsehkamera 5 lenkt. Im Fernsehbild erhält man demgemäß eine Überlagerung des Bildverstärker-Ausgangsbildes und des Abbildes der in der Ebene 16 leuchtenden, von den Enden der Lichtwellenleiter 26a gebildeten Bildelemente. Dieses Abbild stellt die mit Hilfe der Auswahlschaltung 22 gewählte Dominante dar.

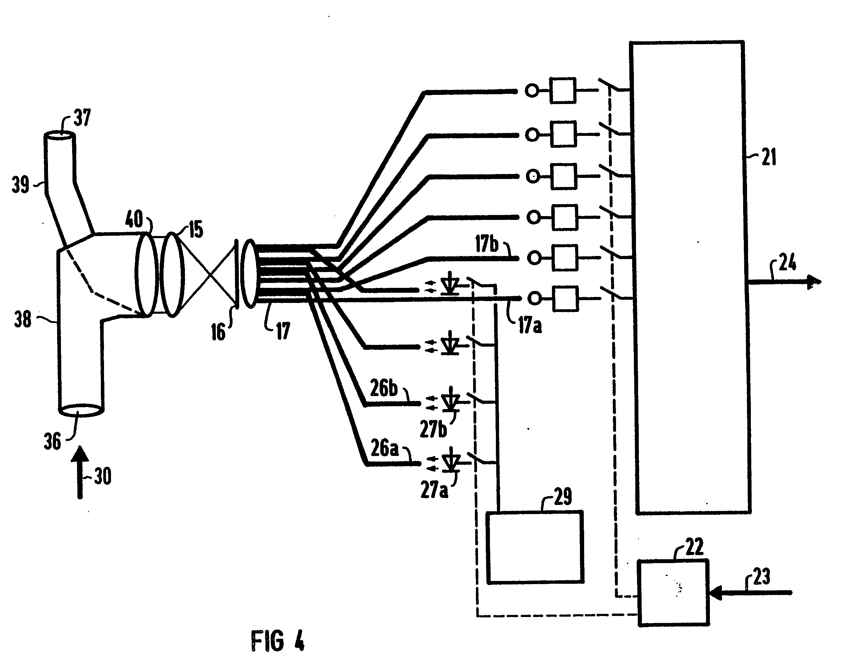

Bei der Variante gemäß Fig. 4 wird anstelle der Spiegel 31 und 32 ein Lichtfaserbündel verwendet, das von einem gemeinsamen Ende 40 von der Optik 15 ausgeht und sich in zwei Teilbündel 38 und 39 im telezentrischen Strahlengang verzweigt. Das Teilbündel 38 empfängt mit seiner Eingangsöffnung einen Teil des Bildverstärker-Ausgangslichtes und leitet ihn weiter über die Optik 15 zu den aktiven Lichtwellenleitern 17a usw. Die Lichtwellenleiter 26a usw. strahlen über die Optik 15 und das Ende 40 in das Teilbündel 39, das dieses Licht mit der Öffnung 37 in den telezentrischen Strahlengang in Richtung zur Fernsehkamera 5 leitet. Dadurch ist ebenfalls eine Einblendung der gewählten Dominante in das Fernsehbild möglich.

Es ist auch eine Kombination einer herkömmlichen Multiplieroptik mit mechanisch umschaltbaren Dominanten mit einer davon getrennten Dominantenanzeige über ein Faserbündel wie 39 möglich. Dabei kann das Abbild der Dominante in der Ebene 16 auch durch eine andere Anordnung erzeugt werden, z.B. durch eine transparente LCD-Matrix anstelle der Kombination der Komponenten 27a und 26a, wobei die Lichtstärkensteuerung 29 und die Schaltvorrichtung 28 beibehalten werden.

## Ansprüche

1. Röntgendiagnostikanlage mit einer Bildverstärker-Fernsehkette (4 bis 7) und Mitteln, die das Ausgangsbild des Röntgenbildverstärkers (4) zusätzlich zur Fernsehkamera (5) erfassen, **gekennzeichnet durch** eine Faseroptik (17), deren eines Ende das Bildverstärker-Ausgangsbild im telezentrischen Strahlengang zwischen Bildverstärker-Ausgangsschirm und Fernsehkamera (5) aufnimmt und die dieses Bild weiterleitet.

2. Röntgendiagnostikanlage nach Anspruch 1, **dadurch gekennzeichnet,** daß den Lichtwellenleitern (17a usw.) der Faseroptik (17) auf der Ausgangsseite individuelle lichtelektrische Wandler (18a usw.) zugeordnet sind.

3. Röntgendiagnostikanlage nach Anspruch 2, **dadurch gekennzeichnet,** daß eine Schaltvorrichtung (20) zur Auswahl vorbestimmter Wandler (18a usw.) für die Bildung eines der mittleren Bildhelligkeit in einem vorbestimmten Bereich entsprechenden Signals vorhanden ist.

4. Röntgendiagnostikanlage nach Anspruch 1, **dadurch gekennzeichnet,** daß den Lichtwellenleitern (17a usw.) der Faseroptik (17) auf der Ausgangsseite eine Betrachtungsoptik (43) zugeordnet ist.

5. Röntgendiagnostikanlage nach Anspruch 3, **dadurch gekennzeichnet,** daß in der Faseroptik (17) ein passives Lichtwellenleiterbündel angeordnet ist, dessen Lichtwellenleiter (26a usw.) auf der Ausgangsseite individuell schaltbare Lichtquellen (27a usw.) zum Einblenden der gewählten Dominante in das Fernsehbild zugeordnet sind.

6. Röntgendiagnostikanlage nach Anspruch 2, **dadurch gekennzeichnet,** daß die Auswertung der Signale der Lichtwellenleiter (17a usw.), die dem äußeren Rand des Bildverstärker-Ausgangsbildes zugeordnet sind, zur Steuerung einer Strahlenblende (41) dienen, die der Überstrahlung des Bildverstärkers (4) durch Einblendung entgegenwirkt.

FIG 1

FIG 2

EP 0 372 101 A1

88 P 3 5 2 8 E

88 P 3528 E

FIG 3

FIG 4

EP 0 372 101 A1

88 P 3 5 2 8 E

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 217 456 (N.V. PHILIPS' GLOEILAMPENFABRIEKEN) * Spalte 1, Zeilen 1-54; Spalte 4, Zeile 45 - Spalte 5, Zeile 46; Spalte 6, Zeilen 6-33; Spalte 7, Zeilen 1-8; Figur 1 * | 1 | H 05 G 1/64 H 04 N 5/32 |
| Y | | 2,3,6 | |
| Y | FR-A-2 476 949 (THOMSON-CSF) * Seite 1, Zeile 16 - Seite 2, Zeile 10; Seite 3, Zeile 20 - Seite 4, Zeile 9; Figuren 2a,2b * | 1,2,4 | |
| Y | US-A-3 912 936 (A.L. CUNNINGHAME et al.) * Spalte 2, Zeile 52 - Spalte 3, Zeile 9; Spalte 4, Zeilen 30-47; Spalte 5, Zeilen 7-59; Figur 1 * | 1,4,6 | |
| A | DE-A-3 225 061 (SIEMENS AG) * Seite 1, Zeilen 3-27; Seite 3, Zeilen 9-31; Figuren 1,2 * | 1,3 | |
| A | EP-A-0 087 843 (N.V. PHILIPS' GLOEILAMPENFABRIEKEN) * Seite 1, Zeile 26 - Seite 3, Zeile 14; Seite 5, Zeile 2 - Seite 6, Zeile 18; Figuren 1,2 * | 1,5 | RECHERCHIERTE SACHGEBIETE (Int. Cl.5) H 04 N H 05 G |
| A | EP-A-0 038 666 (TECHNICARE CORP.) * Seite 3, Zeilen 12-32; Seite 6, Zeile 23 - Seite 7, Zeile 28; Figur 1 * -/- | 1 | |

Der vorliegende Recherchenbericht wurde für alle Patentansprüche erstellt

| Recherchenort | Abschlußdatum der Recherche | Prüfer |
|---|---|---|
| DEN HAAG | 07-08-1989 | HORAK G.I. |

Europäisches
Patentamt

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | PATENT ABSTRACTS OF JAPAN, Band 9, Nr. 43 (E-298)[1766], 22. Februar 1985; & JP-A-59 183 580 (HITACHI MEDEIKO K.K.) 18-10-1984 * Zusammenfassung * ----- | 1,5 | |
| | | | RECHERCHIERTE SACHGEBIETE (Int. Cl.5) |

Der vorliegende Recherchenbericht wurde für alle Patentansprüche erstellt

| Recherchenort | Abschlußdatum der Recherche | Prüfer |
|---|---|---|
| DEN HAAG | 07-08-1989 | HORAK G.I. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
.............................................................
& : Mitglied der gleichen Patentfamilie, übereinstimmendes Dokument

EPO FORM 1503 03.82 (P0403)